# EUROPEAN PATENT APPLICATION

(11) **EP 3 581 202 A1**
(43) Date of publication of application: **18.12.2019**
(21) Application number: 18177686.5
(22) Date of filing: 14.06.2018
(51) Int. Cl.: A61K 39/12, A61K 9/00, A61K 38/06, A61K 31/00, A61K 31/047, A61K 31/198, A61K 31/7016, C08L 29/04

(54) **COMPOSITION AND METHOD FOR STABILISING VACCINES IN A SOLID DOSAGE FORMAT**

(71) Applicant: University College Cork-National University of Ireland, Cork, Cork (IE)
(72) Inventor: Donadei, Agnese, Cork (IE); Flynn, Olivia, Cork (IE); Moore, Anne, Cork (IE)
(74) Representative: Murgitroyd & Company

(57) **Abstract**

A composition for stabilising a vaccine in a solid dosage format is provided wherein the composition comprises an antioxidant, such as glutathione, a monosaccharide or disaccharide sugar, such as trehalose, a polyol sugar, such as sorbitol, one or more salts, such as magnesium chloride and sodium glutamate, and a vaccine. The composition may also comprise an aqueous soluble polymer, such as polyvinyl alcohol (PVA). A preferred composition comprises 40 mM glutathione, 20% w/v trehalose, 3% w/v sorbitol, 1.5% w/v PVA, 3% w/v magnesium chloride and 3% w/v sodium glutamate. Also provided is a method of stabilising a vaccine in a solid dosage format, the method comprising drying the stabilising composition to provide the vaccine in the solid dosage format. The composition and method may be used to stabilise any suitable vaccine, such as poliovirus, in a solid dosage format, such as microneedle patches.

## Description

### Field of the Invention

The present invention relates to a composition and method for stabilising vaccines, in particular, for stabilising vaccines in a solid dosage format.

### Background of the Invention

Vaccination is the most effective method of preventing infectious diseases. Widespread immunity due to vaccination is largely responsible for the worldwide eradication of smallpox and the restriction of diseases such as polio, measles, and tetanus from much of the world. The World Health Organization (WHO) reports that licensed vaccines are currently available for twenty-five different preventable infections. Vaccines are often delivered by injection requiring administration by a trained health worker and use of sterile syringes. This results in hazardous waste as disposal of used needles, syringes and glass vials is required. Furthermore, vaccines are labile biologics. Vaccines in liquid dosage format require costly cold chain storage and distribution and reconstitution in some cases. These logistic costs are important economic limiting factors.

The polio vaccine is used to immunise against poliomyelitis (polio), which is an infectious disease caused by poliovirus strains type 1, type 2 and type 3. The polio vaccine is part of national routine immunization schedules and polio immunization campaigns have almost globally eradicated these three strains. Two vaccine strategies have been used to generate this successful outcome. Oral polio vaccines (OPV), developed by Sabin, are administered by drops into the mouth and are relatively inexpensive to purchase and administer. However, this is a live attenuated vaccine and on rare occasions can cause vaccine-associated paralytic poliomyelitis. Inactivated (killed) polio vaccines (IPV), developed by Salk, involve the production of infectious poliovirus, which is then inactivated and formulated for systemic administration with a needle-and-syringe. IPV is currently given by intramuscular injection. As such, it needs to be administered by a trained health worker and sterile syringes are required. IPV is related to hazardous waste requiring disposal of used needles, syringes and glass vials. Although IPV is one of the safest vaccines in use, there are increased bio-safety concerns at the point of manufacture. This is largely responsible for the higher cost of this vaccine. As part of the WHO's Global Polio Eradication and Endgame Strategic Plan, an action plan is being implemented to replace OPV with IPV and to use safer Sabin strains to manufacture inactivated polio vaccine, termed Sabin IPV (sIPV). The switch from OPV to IPV will require significant increases in cost and complexity of immunization, as vaccinators will need to administer the vaccine by injection rather than oral drops. Moreover, due to planned changes in the composition of polio vaccines from OPV to IPV to sIPV and from trivalent to bivalent vaccines, and given the cost of cold chain storage, vaccine manufacturers are reluctant to over-produce vaccines that may be surplus to requirements and are de-risking the costs and resource implication of cold chain storage of polio vaccines. This has led to global shortages of polio vaccines for routine immunization and for stockpiling in case of an outbreak.

Therefore, a solution is needed to the problems of (i) increased cost and complexity of injection-based immunization with vaccines such as IPV and (ii) expensive cold chain storage and distribution which makes stockpiling vaccines, such as polio vaccines, difficult.

Previous efforts to stabilise the polio vaccine have focused on stabilising the polio vaccine in a liquid form or by lyophilising the vaccine (sIPV) to create a solid form. This freeze-drying method relies on complex freeze-dry cycles and defined parameters to form a vaccine "cake". Furthermore, specialist lyophilisation equipment is required.

### Summary of the Invention

According to a first aspect of the present invention, there is provided a vaccine composition for stabilising a vaccine in a solid dosage format, wherein the vaccine composition comprises, consists essentially of or consists of:
- an antioxidant;
- a monosaccharide or disaccharide sugar;
- a polyol sugar;
- one or more salts; and
- a vaccine.

According to a second aspect, there is provided a vaccine in a solid dosage format comprising, consisting essentially of, or consisting of a vaccine composition according to a first aspect of the present invention, wherein the vaccine composition has been dried to provide the vaccine in the solid dosage format.

According to a third aspect of the present invention, there is provided a method of stabilising a vaccine in a solid dosage format, the method comprising the steps of:
- providing a vaccine composition according to a first aspect of the present invention; and
- drying the vaccine composition to provide the vaccine in the solid dosage format.

According to a fourth aspect of the present invention, there is provided use of a vaccine composition according to a first aspect of the present invention to stabilise a vaccine in a solid dosage format.

### Detailed Description of the Invention

The present inventors have developed a composition and method for stabilising vaccines that can be used to provide vaccines in a solid dosage format without substantial loss of vaccine potency. The provision of a vaccine in a solid dosage format is advantageous as providing a non-injection based vaccine dosage format overcomes the problems inherent in injection-based immunization. The provision of solid dosage formats, such as patches for buccal, sublingual or skin administration, eliminates the requirement for vaccine compositions to be injected. This allows for alternative routes of administration for vaccination, such as polio vaccination. The invention thus opens up feasibility to oral, intradermal and mucosal vaccine administration using capsules, microneedles and powders. Oral vaccines have been shown to be effective when vaccinations were administered by volunteer staff without formal training and there is no risk of blood contamination. Microneedles use pointed projections fabricated into arrays that can create vaccine delivery pathways through the skin. This dermal administration potentially increases the effectiveness of vaccination, while requiring less vaccine than injection

Furthermore, vaccines in solid dosage format commonly are more stable and less prone to damage or to spoilage by thermal stresses in transport and storage. Such stability reduces the need for cold chain storage and distribution which is often required in order to keep vaccines within a restricted temperature range from the manufacturing stage to the point of administration. This, in turn, may decrease costs of vaccines. The composition and method of the present invention both chemically and physically stabilise vaccines, particularly whole inactivated vaccines, in solid dosage formats such that the vaccines can be stored, handled and used at room temperature. Thermostabilising the vaccine so that cold chain logistics are eliminated allows vaccine stockpiling in regular drug distribution systems. This would have a significant impact on solving vaccine storage and distribution. The use of a solid dosage format for vaccination thus allows an effective vaccination in the absence of refrigerated storage conditions, reducing costs and increasing vaccine stability in unfavourable environmental conditions. For example, in polio endemic countries, where cold chain is a significant issue in vaccine distribution, improved stability would assist in an increased availability of vaccine stocks for potential disease outbreaks and more effective vaccination.

The antioxidant may comprise one or more amino acids, such as sulphur-containing amino acids. The antioxidant may be selected from the group consisting of cysteine, methionine, tryptophan, taurine, glutathione, glycine, glutamic acid, lipoic acid, N-acetylcysteine, ascorbic acid (vitamin C) and combinations thereof. In certain embodiments, the antioxidant may be selected from the group consisting of glutathione, vitamin C, cysteine, glycine, glutamic acid and a combination comprising cysteine (Cys), glutamic acid (Glu) and glycine (Gly). The antioxidant may be glutathione. The antioxidant (e.g. glutathione, vitamin C, cysteine, glycine, glutamic acid or a combination comprising cysteine (Cys), glutamic acid (Glu) and glycine (Gly)) may be present within the composition at a concentration of 20 to 80, 30 to 50 or 25 to 35 mM. The antioxidant may be present within the composition at a concentration of 20, 25, 30, 35, 40, 45, 50, 55, 60, 65, 70, 75 or 80 mM. The antioxidant (e.g. glutathione) may be present within the composition at a concentration of 40 mM. The inventors have shown that vitamin C may be used in place of glutathione. The inventors have also shown that cysteine, glycine, glutamic acid or a combination comprising all three may be used in place of glutathione.

The monosaccharide or disaccharide sugar may be any suitable monosaccharide or disaccharide sugar. The monosaccharide sugar may be selected from glucose or galactose. The disaccharide sugar may be selected from the group consisting of trehalose, sucrose, lactose, maltose and combinations thereof. The disaccharide sugar may be trehalose. The monosaccharide or disaccharide sugar (e.g. trehalose) may be present within the composition at a concentration of 10 to 40% w/v, 10 to 25% w/v, 10 to 20% w/v, 15 to 25% w/v, 15 to 30% w/v or 15 to 20% w/v. The monosaccharide or disaccharide sugar (e.g. trehalose) may be present within the composition at a concentration of 10%, 15%, 20%, 25%, 30%, 35% or 40% w/v. The monosaccharide or disaccharide sugar (e.g. trehalose) may be present within the composition at a concentration of 15% w/v or 20% w/v.

The polyol sugar may be selected from the group consisting of sorbitol, mannitol, maltitol, lactitol, xylitol, iosmalt, erythritol and combinations thereof. The polyol sugar may be sorbitol. The polyol sugar has a stabilising effect on the vaccine. The polyol sugar (e.g. sorbitol) may be present within the composition at a concentration of 3-8% w/v, 3-7% w/v, 3-6% w/v or 3-5% w/v. The polyol sugar (e.g. sorbitol) may be present at a concentration of 3% w/v, 4% w/v, 5% w/v, 6% w/v, 7% w/v or 8% w/v. The polyol sugar (e.g. sorbitol) may be present at a concentration of 3% w/v or 5% w/v. The inventors have shown that a polyol sugar (e.g. sorbitol) is required for stability of IPV.

The composition may comprise an aqueous soluble polymer. The aqueous soluble polymer may be a commercially available aqueous soluble polymer. For example, it may be selected from the group consisting of polyvinyl alcohol (PVA), carboxymethyl cellulose (CMC), polyvinyl pyrrolidone (PVP), hydroxypropyl methylcellulose (HPMC), ethylcellulose, polyacrylamide, polyacrylic acids, polyacrylates, acrylic/maleic copolymers, and combinations thereof. Typically, the aqueous soluble polymer may be PVA. The aqueous soluble polymer provides the composition with mechanical strength, in particular, where the vaccine is being provided in dissolving microneedle patches. The aqueous soluble polymer (e.g. PVA) may be present within the composition at a concentration of 0% to 6% weight/volume (w/v), 0% to 5.5% w/v or 0% to 5% w/v. The concentration may be 5% w/v or less, 4% w/v or less, or 3.5% w/v or less, or 3% w/v or less. The inventors have found that an optimal PVA concentration is 3% w/v or less. Accordingly, the aqueous soluble polymer (e.g. PVA) may be present within the composition at a concentration of 3% w/v or less, 2.5% w/v or less, 2% w/v or less, 1.5% w/v or less, 1% w/v or less or 0.5% w/v or less. The concentration of the aqueous soluble polymer (e.g. PVA) may be 1.5% w/v.

The composition comprises one or more salts, such as magnesium chloride, sodium glutamate or a combination thereof. The salt has a stabilising effect on the vaccine. The one or more salts (e.g. magnesium chloride, sodium glutamate or a combination thereof) may be present within the composition at a concentration of 0.5-8% w/v, 1-7% w/v, 1-6 % w/v, 1-5% w/v, 2-4% w/v, 2.5 - 3.5% w/v or 3% w/v. The composition may comprise magnesium chloride at 3% w/v and/or sodium glutamate at 3% w/v. The composition may comprise both magnesium chloride and sodium glutamate each at 2.5% to 3.5% w/v, preferably 3% w/v.

In certain embodiments, the composition includes one or more of the following:
a) a combination of glutathione and sorbitol;
b) a combination of glutathione and trehalose;
c) a combination of glutathione and salt;
d) a combination of glutathione and poly-vinyl alcohol (PVA);
e) a combination of sorbitol and trehalose;
f) a combination of sorbitol and salt;
g) a combination of sorbitol and poly-vinyl alcohol; and
h) a combination of all of the above.

In certain embodiments, the composition comprises, or consists essentially of:
- glutathione, vitamin C, glycine, cysteine, glutamic acid or a combination of glycine, cysteine and glutamic acid as the antioxidant;
- trehalose as the disaccharide sugar;
- sorbitol as the polyol sugar; and
- magnesium chloride and/or sodium glutamate as the one or more salts.

In certain embodiments, the composition further includes PVA as the aqueous soluble polymer.

In certain embodiments, the composition comprises, consists essentially of, or consists of:
- antioxidant (e.g. glutathione, vitamin C, glycine, cysteine, glutamic acid or a combination of glycine, cysteine and glutamic acid) at a concentration of 20 to 80 mM, optionally 40 mM;
- monosaccharide or disaccharide sugar (e.g. trehalose) at a concentration of 10 to 40% w/v, optionally 20% w/v;
- polyol sugar (e.g. sorbitol) at a concentration of 3-8% w/v, optionally 3% w/v;
- an aqueous soluble polymer (e.g. PVA) at a concentration of 0-5% w/v, optionally 3% w/v or less (e.g. 1.5% w/v); and
- one or more salts (e.g. magnesium chloride and/or sodium glutamate) each at a concentration of 0.5-5% w/v, optionally 3% w/v.

In certain embodiments, the composition comprises, consists essentially of, or consists of:
- 40 mM glutathione;
- 20% w/v trehalose;
- 3% w/v sorbitol;
- 1.5% w/v PVA;
- 3% w/v magnesium chloride; and
- 3% w/v sodium glutamate.

The composition may also include a buffer. A buffer may be used to provide the required pH if necessary, for example for stability of the aqueous soluble polymer or the antioxidant. For example, PVA is stable at a pH of between 6 and 7.4.

The composition may be used to provide stability for monovalent, bivalent and trivalent vaccines in a solid dosage format. The vaccine may be a vaccine in a dosage format other than a solid dosage format, e.g. a liquid form. The vaccine may be for polio, measles, mumps, German measles, chicken pox, hepatitis, herpes, or mixtures of these. The vaccine may comprise any suitable type of vaccine, such as live attenuated vaccines, inactivated vaccines, subunit vaccines, whole vaccines, whole inactivated vaccines, recombinant vaccines, polysaccharide vaccines, conjugate vaccines, toxoid vaccines, vector vaccines, virus-like particle (VLP) vaccines and adenovirus based vaccines. In particular, the vaccine may comprise an inactivated virus. The virus may be any virus suitable for use in a vaccine. The virus may be a picornavirus. The picornavirus may be selected from the group consisting of enterovirus, aphthovirus, cardiovirus, rhinovirus and hepatovirus genera. In particular, the picornavirus may be an enterovirus. Typically the enterovirus may be poliovirus. The apthovirus may cause foot and mouth disease. Where the virus is poliovirus, the poliovirus may be selected from Salk IPV (conventional IPV) and Sabin IPV (sIPV). The poliovirus may be type 1, 2 or 3, for example, Salk type 1, 2 or 3 or Sabin type 1, 2 or 3.

The composition may comprise one or more adjuvants, stabilizers or preservatives. Adjuvants enhance the immune response of the antigen, stabilizers increase the storage life, and preservatives allow the use of multidose vials. In particular, the composition may comprise one or more excipients selected from the group consisting of aluminium salts or gels, antibiotics, formaldehyde, monosodium glutamate, 2-phenoxyethanol,

The vaccine composition comprising the vaccine (e.g. the vaccine in liquid format) may be dried to provide the vaccine in a solid dosage format. The vaccine composition may be dried at ambient temperature or any temperature suitable for use with thermosensitive biological materials. Lyophilisation is not essential for drying. The solid dosage format may be selected from the group consisting of microneedles, powders, capsules, wafers, microneedle patches (e.g. dissolving microneedle patches) and patches, such as patches for buccal, sublingual or skin administration. Typically the solid dosage format is not a foam. The vaccine in solid dosage format may be administered by any suitable means of administration, such as, by oral or intradermal administration. As such, the requirement to administer by injection is eliminated.

In the method of stabilising a vaccine in a solid dosage format, providing a vaccine composition may comprise preparing the composition for stabilising a vaccine in a solid dosage format by combining excipients as described herein and a vaccine. The vaccine may be in a liquid format or any other format other than a solid dosage format and may be any suitable vaccine, for example, existing polio vaccines and other types of vaccines.

Drying may comprise using a vacuum. This accelerates drying. The method does not require lyophilisation. The vaccine composition is dried until only a small amount of residual moisture remains in the vaccine composition. The appropriate or preferred amount of residual moisture depends on the type of vaccine. This may be determined by those of skill in the art by consulting appropriate literature. In certain cases, the vaccine composition (e.g. a vaccine against poliovirus) is dried until the residual moisture is 3% or less or 1% or less (J.C. May, et al. Measurement of final container residual moisture in freeze-dried biological products (Dev. Biol. Stand., 74 (1992), pp. 153-164)). Generally, the lower amount of residual moisture remaining, the better, although some level of residual moisture may be needed. The appropriate drying time for a particular vaccine may be determined by those of skill in the art. The vaccine (e.g. a vaccine against poliovirus) may be dried for a time period ranging from 24 hours to 48 hours, typically 36 hours. The method of the invention merely relies on drying at suitable (e.g. ambient) temperature or by vacuum to accelerate drying. Crucially, it can be independent of lyophilisation.

The composition and method of the invention can be used to produce a solid dosage format that stabilises viruses, such as inactivated polio virus vaccine (IPV). The stabilising composition and method can be used for monovalent, bivalent and trivalent vaccines. The stabilising composition and method can be used with both sIPV and conventional IPV and can be used to prevent poliomyelitis caused by poliovirus strains type 1, type 2 and type 3.

The present invention thus addresses two problems in the vaccine field, in particular, the polio field, firstly the issue of costs and logistics surrounding injection-based immunisation and secondly the issue of vaccine stability and cold storage.

The vaccine in solid dosage format may be administered to a subject in need thereof, e.g. a subject who is at risk of being infected by the virus in question. The vaccine in solid dosage format may be administered to the subject via any suitable route. In particular, the vaccine in solid dosage format may be administered orally, by mucosal routes or topically. The vaccine may be administered via microneedles, patches, wafers or any other suitable solid dosage format. The vaccine in solid dosage format may be administered by a non-medically trained person. The vaccine in solid dosage format may be stored and transported at room temperature.

The composition is formulated such that at least 50% of the potency of the vaccine prior to drying is retained in the solid dosage format. Typically, at least 60%, 70%, 80%, 85%, 90%, 95%, 96%, 97%, 98% or 99% of vaccine potency is retained. For IPV, potency may be measured in terms of D-antigen activity.

### Definitions

Unless otherwise defined, all technical and scientific terms used herein have the meaning commonly understood by a person who is skilled in the art in the field of the present invention.

An aqueous soluble polymer may also be referred to as a water soluble polymer and is used herein to describe polymers that dissolve, disperse or swell in water.

The term "polyvinyl alcohol" or "PVA" as used herein is intended to refer to all types of PVA. As such, the PVA may be short chain PVA, long chain PVA, low molecular weight PVA or high molecular weight PVA.

The term "antioxidant" as used herein refers to a substance that inhibits oxidation.

The term "monosaccharide sugar" refers to a sugar that cannot be hydrolysed to give a simpler sugar. The term "disaccharide sugar" refers to a sugar which is former when two monosaccharides are joined by glycosidic linkage.

The term "polyol sugar" refers a sugar from the class of polyol sugars. This term encompasses sugar alcohols.

As used herein, "stabilising" a vaccine refers to reducing or preventing loss of potency of the vaccine. Preferably, the composition and method of the invention both chemically and physically stabilise vaccines in solid dosage formats. This allows the vaccines to be stored, handled and used at room temperature. "Stabilising" may also be understood as retaining the antigenicity and/or immunogenicity of a virus in a vaccine. For IPV, potency may be measured by quantitation of D antigen content and recovery efficiency can be measured by comparing values before and after preparing the solid dosage format. Quantitation of D antigen content may be carried out using ELISA. "Recovery efficiency" as used herein means the percentage of the D-antigen activity from an initial vaccine stock that was recovered after preparing the solid dosage format.

The words "comprises/comprising" and the words "having/including" when used herein with reference to the present invention are used to specify the presence of stated features, integers, steps or components, but do not preclude the presence or addition of one or more other features, integers, steps, components or groups thereof. The term "consists essentially of" as used herein means that no other components that materially affect the stabilising composition are present. This does not rule out the presence of additional minor components.

As used herein, terms such as "a", "an" and "the" include singular and plural referents unless the context clearly demands otherwise.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### Example 1 - Stabilising Compositions for Poliovirus in a Solid Dosage Format

The formulations shown in Tables 1, 2 and 3 were assessed for recovery efficiency. Initial vaccine stock was added to the formulation. This was then dispensed into microneedle moulds, dried and resuspended in buffer. The D-antigen content was analysed using ELISA. A 70% "recovery efficiency", for example, means that 70% of the D-antigen activity from the initial vaccine stock was recovered. A recovery of 50% was considered the limit of acceptability. Results for Salk type 3 and a drying time of 24 hours are shown in Tables 1 and 2. All reported excipients concentrations refer to the final formulation volume in the presence of the vaccine stock and as such can be considered as final concentrations.

**Table 1. Formulations using Salk type 3 and a drying time of 24 hours**

| | | | | | | | | | |
|---|---|---|---|---|---|---|---|---|---|
| Trehalose (% w/v) | 15 | 15 | 15 | 15 | 15 | 25 | 15 | 15 | 15 |
| Sorbitol (% w/v) | 5 | 5 | 5 | 5 | 0 | 0 | 5 | 5 | 5 |
| Sodium Glutamate (% w/v) | 3 | 3 | 3 | 3 | 3 | 3 | 3 | 3 | 3 |
| MgCl2 (% w/v) | 3 | 3 | 3 | 3 | 3 | 3 | 3 | 3 | 3 |
| PVA (% w/v) | 5 | 0 | 3 | 0 | 3 | 3 | 5 | 3 | 3 |
| Glutathione (mM) | 20 | 20 | 40 | 40 | 40 | 40 | 40 | | |
| Vitamin C (mM) | | | | | | | | 40 | |
| Cys+Glu+Gly (mM) | | | | | | | | | 40 |
| Cys (mM) | | | | | | | | | |
| Glycine (mM) | | | | | | | | | |
| Glutamic Acid (mM) | | | | | | | | | |
| % of initial/stock IPV recovered | 46% | 70% | 70% | 64% | 23% | 39% | 46% | 73% | 46% |

**Table 2. Formulations using Salk type 3 and a drying time of 24 hours**

| | | | | | | | | |
|---|---|---|---|---|---|---|---|---|
| Trehalose (% w/v) | 15 | 15 | 15 | 15 | 15 | 15 | 15 | 15 |
| Sorbitol (% w/v) | 5 | 5 | 5 | 5 | 5 | 5 | 5 | 5 |
| Sodium Glutamate (% w/v) | 3 | 3 | 3 | 3 | 3 | 3 | 3 | 3 |
| MgCl2 (% w/v) | 3 | 3 | 3 | 3 | 3 | 3 | 3 | 3 |
| PVA (% w/v) | 3 | 3 | 3 | 5 | 5 | 5 | 5 | 5 |
| Glutathione (mM) | | | | | | | | |
| Vitamin C (mM) | | | | 40 | | | | |
| Cys+Glu+Gly (mM) | | | | | 40 | | | |
| Cys (mM) | 40 | | | | | 40 | | |
| Glycine (mM) | | 40 | | | | | 40 | |
| Glutamic Acid (mM) | | | 40 | | | | | 40 |
| % of initial/stock IPV recovered | 51% | 60% | 59% | 46% | 51% | 67% | 66% | 63% |

The results shown in Tables 1 and 2 indicate that an optimal concentration of PVA is less than 3%. An optimal glutathione concentration range is 20-80 mM. Results for vitamin C were equivalent to glutathione. Results for cysteine, glutamic acid, glycine or a combination comprising all three were equivalent. Sorbitol in a range of 3-8% w/v was found to be essential for IPV.

Based on the results observed, the inventors provided a preferred final formulation having the excipients shown in Table 3.

**Table 3. Final Formulation using Salk type 1, 2 or 3 and a drying time of 36 hours**

| | |
|---|---|
| Trehalose (% w/v) | 20 |
| Sorbitol (% w/v) | 3 |
| Sodium Glutamate (% w/v) | 3 |
| MgCl2 (% w/v) | 3 |
| PVA (% w/v) | 1.5 |
| Glutathione (mM) | 40 |
| Recovery efficiency TYPE 1 | 110% |
| Recovery efficiency TYPE 2 | 100% |
| Recovery efficiency TYPE 3 | 85% |

The final formulation as shown in Table 3 provided surprisingly high IPV recovery efficiency values of 110%, 100% and 85% for Salk types 1, 2 and 3 respectively. As such, the composition and method of the invention assist in preserving D-antigen content during formation of the solid dosage format such that there is no loss in the potency of the vaccine. In some cases, an increase in D antigen content was observed indicating a surprising increase in potency.

### Example 2 - Microneedles

The final formulation of Table 3 containing Salk IPV type 1-2-3 was incorporated into dissolvable microneedle patches as an example of a potential solid-state vaccine administration platform. These were shown to exhibit strong mechanical robustness, as exemplified by mechanical strength and skin penetration of the dissolvable microneedle patches containing the IPV in the final formulation.

## Claims

1. A vaccine composition for stabilising a vaccine in a solid dosage format, the vaccine composition comprising:
- an antioxidant;
- a monosaccharide or disaccharide sugar;
- a polyol sugar;
- one or more salts;
- a vaccine; and
- optionally an aqueous soluble polymer.

2. The vaccine composition as claimed in claim 1, wherein the vaccine composition comprises:
- 20 to 80 mM antioxidant;
- 10 to 40% w/v monosaccharide or disaccharide sugar;
- 3-8% w/v polyol sugar;
- 0.5-8% w/v salts; and
- 0-5% w/v aqueous soluble polymer.

3. The vaccine composition as claimed in claim 1 or 2, wherein:
- the antioxidant is selected from the group consisting of glutathione, vitamin C, glycine, cysteine, glutamic acid and a combination of glycine, cysteine and glutamic acid;
- the monosaccharide or disaccharide sugar is the disaccharide sugar trehalose;
- the polyol sugar is sorbitol;
- the aqueous soluble polymer is polyvinyl alcohol (PVA); and/or
- the one or more salts comprise magnesium chloride and sodium glutamate.

4. The vaccine composition as claimed in any one of claims 1 to 3, wherein the vaccine composition comprises:
- 20 to 80 mM antioxidant selected from the group consisting of glutathione, vitamin C, glycine, cysteine, glutamic acid and a combination of glycine, cysteine and glutamic acid;
- 10 to 40% w/v trehalose;
- 3-8% w/v sorbitol;
- 0-5% w/v PVA;
- 0.5-5% w/v magnesium chloride; and
- 0.5-5% w/v sodium glutamate.

5. The vaccine composition as claimed in any one of claims 1 to 4, wherein the vaccine composition comprises:
- 40 mM glutathione;
- 20% w/v trehalose;
- 3% w/v sorbitol;
- 1.5% w/v PVA;
- 3% w/v magnesium chloride; and
- 3% w/v sodium glutamate.

6. The vaccine composition as claimed in any one of claims 1 to 5, wherein the vaccine composition is dried to provide the vaccine in a solid dosage format.

7. A method of stabilising a vaccine in a solid dosage format, the method comprising the steps of:
- providing a vaccine composition as claimed in any one of claims 1 to 5; and
- drying the vaccine composition to provide the vaccine in the solid dosage format.

8. The method as claimed in claim 7, wherein providing a vaccine composition comprises providing a vaccine composition as claimed in claim 5.

9. The method as claimed in claim 7 or 8, wherein drying the vaccine composition to provide the vaccine in the solid dosage format does not comprise lyophilisation.

10. Use of a vaccine composition according to any one of claims 1 to 5 for stabilising a vaccine in a solid dosage format.

11. The vaccine composition as claimed in claim 6, the method as claimed in any one of claims 7 to 9, or the use as claimed in claim 10, wherein the solid dosage format is selected from the group consisting of microneedles, powders, capsules, wafers, microneedle patches and patches.

12. The vaccine composition, method or use as claimed in claim 11 wherein the solid dosage format is microneedle patches.

13. The vaccine composition as claimed in any one of claims 1 to 6, 11 or 12, the method as claimed in any one of claims 7 to 9, 11 or 12, or the use as claimed in any one of claims 10 to 12, wherein the vaccine comprises a picornavirus.

14. The vaccine composition, method or use as claimed in claim 13, wherein the picornavirus is poliovirus.

15. The vaccine composition, method or use as claimed in claim 14, wherein the poliovirus is Salk Inactivated Polio Vaccine (IPV) or Sabin IPV (sIPV).
